# EUROPÄISCHE PATENTSCHRIFT

(11) **EP 0 775 690 B1**
(45) Veröffentlichungstag und Bekanntmachung des Hinweises auf die Patenterteilung: **20.06.2001**
(21) Anmeldenummer: 96118447.0
(22) Anmeldetag: 18.11.1996
(51) Int. Cl.: C07C 231/12, C07C 233/47, C07B 41/12

(54) **Verfahren zur Herstellung von N-Lauroyl-L-glutaminsäuredimethylester**
Process for the preparation of N-lauroyl-L-glutamic acid dimethylester
Procédé pour la préparation de l'ester diméthylique de l'acide N-lauroyl-L-glutamique

(30) Priorität: 24.11.1995 DE 19543792
(43) Veröffentlichungstag der Anmeldung: 28.05.1997
(73) Patentinhaber: Clariant GmbH, 65929 Frankfurt am Main (DE)
(72) Erfinder: Engelhardt, Fritz, Dr., Chesapeake, Virginia 23320 (US); Müller, Manfred, 63571 Gelnhausen (DE); Wessling, Michael, Dr., 63477 Maintal-Bischofsheim (DE)

(56) Entgegenhaltungen:
- INDIAN JOURNAL OF CHEMISTRY, SECTION B, Bd. 15, Nr. 8, 1977, Seiten 766-767, XP002026651 JAIN, J. C. ET AL.: "A new approach for esterification of amino acids."
- "BEILSTEINS HANDBUCH DER ORGANISCHEN CHEMIE, vierte Auflage, drittes Ergänzungswerk, Bd 4, Teil 2, Seite 1545 " 1963 , SPRINGER-VERLAG , BERLIN.GÖTTINGEN.HEIDELBERG, DE XP002026652 * Absatz 2 *
- DATABASE WPI Section Ch, Week 7946 Derwent Publications Ltd., London, GB; Class B05, AN 79-83176B XP002026653 & JP 54 128 513 A (DAI-ICHI KAGAKU YAK) , 5.Oktober 1979
- PATENT ABSTRACTS OF JAPAN vol. 009, no. 247 (C-307), 3.Oktober 1985 & JP 60 105645 A (YASUMITSU TAMURA), 11.Juni 1985,
- PATENT ABSTRACTS OF JAPAN vol. 011, no. 242 (C-438), 7.August 1987 & JP 62 048648 A (FUJISAWA PHARMACEUT CO LTD), 3.März 1987,

## Beschreibung

Die vorliegende Erfindung betrifft ein Verfahren zur Herstellung von N-Lauroyl-L-glutaminsäuredimethylester durch Umsetzung von N-Lauroyl-L-glutaminsäure mit Methanol in Gegenwart von Orthoameisensäuretrimethylester.

N-Lauroyl-L-glutaminsäure-bis-n-butylamid ist ein Grundstoff für die Kosmetikindustrie, zu dessen Herstellung reiner Lauroyl-L-glutaminsäuredimethylester benötigt wird. Allerdings steht bisher kein Verfahren zur Verfügung, das diesen Ester in effizienter Weise in reiner Form liefert. Für die Darstellung von Carbonsäure-Estern und Dicarbonsäure-Diestern gibt es eine Vielzahl von in der Literatur hinlänglich zitierten Syntheserouten. Man kann dabei die verschiedensten funktionellen Gruppen in Ester-Funktionalitäten umwandeln. So gelingt die Ester-Synthese durch Umsetzung aktivierter Carbonsäurederivate wie z. B. Säurechloriden oder Säureanhydriden mit Alkoholen meist in sehr guten bis quantitativen Ausbeuten. Betrachtet man dagegen die direkte Veresterung von Carbonsäuren mit Alkoholen, so handelt es sich um eine typische Gleichgewichtsreaktion. Die Verschiebung des Gleichgewichts zugunsten der Produkte erfolgt bekanntermaßen durch Einsatz eines der Reaktanden im Überschuß. Um die Ausbeuten möglichst quantitativ zu gestalten, ist es in den meisten Fällen - so auch bei der Umsetzung von N-Lauroyl-L-glutaminsäure mit Methanol - nötig, zusätzlich eines der Reaktionsprodukte, zumeist das gebildete Reaktionswasser, aus dem Gleichgewicht zu entfernen. Dazu werden in der Regel Schleppmittel eingesetzt, die es ermöglichen, das Reaktionswasser azeotrop abzudestillieren.

Wird der Ester eines Alkolhols gewünscht, der ein Azeotrop mit Wasser bildet, so kann dieser als Mittel der Wahl eingesetzt werden.
Da Methanol kein Azeotrop mit Wasser bildet, scheidet die letztgenannte Möglichkeit zur Gleichgewichtsverschiebung aus. Die Umsetzung von N-Lauroyl-L-glutaminsäure (LGS) in einem Überschuß an Methanol (5-10 mol MeOH/mol LGS) führt nach längerer Reaktions-zeit (8-10 Stunden) nur zu einem Umsatz von ca.
50-60 % (HPLC). Auch eine Verdoppelung der Reaktionszeit führt zu keiner nennenswerten Verbesserung des Umsatzes.

J. C . Jain et al beschreiben in Indian J. Chem., Vol 15 B (8) 1977, S. 766-767 die Veresterung von Aminosäuren, wobei Orthocarbonsäureester als Wasserfänger zur Entfernung des Reaktionswassers eingesetzt werden.

Überraschenderweise wurde nun gefunden, daß die Umsetzung von LGS zu N-Lauroyl-L-glutaminsäuredimethylester praktisch quantitativ innerhalb sehr kurzer Reaktionszeiten verläuft, wenn man in Gegenwart von Orthoameisensäuretrimethylester unter Säurekatalyse arbeitet.

Die vorliegende Erfindung betrifft demnach ein Verfahren zur Herstellung von N-Lauroyl-L-glutaminsäuredimethylester durch Umsetzung von N-Lauroyl-L-glutaminsäure mit Methanol, dadurch gekennzeichnet, daß in Gegenwart von Orthoameisensäuretrimethylester unter Säurekatalyse gearbeitet wird.

Bevorzugt werden bei dem erfindungsgemäßen Verfahren pro Mol LGS 1 bis 10 Mol Methanol und 0,01 bis 0,2 Mol des Katalysators eingesetzt. Besonders bevorzugt sind 1,5 bis 3 Mol Methanol und 0,03 bis 0,08 Mol Katalysator pro Mol LGS. Als saurer Katalysator wird bevorzugt p-Toluolsulfonsäure eingesetzt, wobei aber auch alle anderen zu diesem Zweck gebräuchlichen Säuren verwendet werden können.

Bei dem erfindungsgemäßen Verfahren reagiert der Orthoameisensäuretrimethylester mit dem bei der Veresterung freigesetzten Wasser zu Ameisensäuremethylester und Methanol ab. In einer bevorzugten Ausführungsform des erfindungsgemäßen Verfahrens wird der bei 31-32°C siedende Ameisensäuremethylester im Augenblick seiner Bildung destillativ aus dem Reaktionsgemisch entfernt, während das gebildete Methanol zur Veresterung der N-Lauroyl-L-glutaminsäure mitverwendet wird.

Das erfindungsgemäße Verfahren verläuft glatt und führt in 3 bis 5 Stunden nach destillativer Entfernung des Methanol-Überschusses in praktisch quantitativer Ausbeute und hoher Reinheit zum gewünschten Produkt.

Die für das erfindungsgemäße Verfahren benötigten Ausgangssubstanzen sind allesamt bekannt und sind im Handel erhältlich.

### Beispiele

### Beispiel 1

In einem 2l-Vierhalskolben mit Thermometer, KPG-Rührer, Tropftrichter und temperierbarer Kolonne mit aufgesetzter Claisen-Brücke werden 1000 g (3.034 mol) LGS in 200 g (6.242 mol) Methanol eingetragen und nach Zugabe von 20 g (0.116 mol) p-Toluolsulfonsäure zum Rückfluß erhitzt (71 °C). Nun tropft man über einen Zeitraum von 3.5 h 670 g (6.314 mol) Orthoameisensäuretrimethylester zu. Sobald die ersten Tropfen zugegeben sind, setzt in der auf 39°C temperierten Kolonne verstärkter Rückfluß ein und nach kurzer Zeit beginnt Ameisensäuremethylester über Kopf abzudestillieren (31-36°C). Nach Zugabe des Orthoesters wird weitere 30 min am Rückfluß gekocht, wobei der Destillatstrom gegen Null geht. Die Destillatmenge entspricht der Theorie (ca. 380 g, ∼ 6.1 mol Ameisensäuremethylester + mitgerissenes Methanol). Man destilliert bei Normaldruck den Überschuß Methanol ab, dann unter Vakuum Reste von Orthoester und erhält nach Abkühlen eine weitgehend farblose Schmelze. Der Fortgang der Reaktion kann dünnschichtchromatographisch verfolgt werden (Produkt: nur ein Fleck). Die Schmelze ergibt nach Zerkleinerung ein farbloses Pulver vom Schmelzpunkt 62-63°C.
- Ausbeute:: 1099.2 g (>98 %) N-Lauroyl-L-glutaminsäuredimethylester
- Reinheit:: DC rein, Startfleck p-TosOH
HPLC (FI.%) >98 %
Verseifungszahl: 313 mg KOH/g Substanz
(Theorie: 314 mg KOH/g)

### Beispiel 2 (Vergleich)

In einem 11-Vierhalskolben mit Thermometer, KPG-Rührer und Rückflußkühler werden 100 g (0.303 mol) LGS in 97.2 g (3.034 mol) Methanol eingetragen und nach Zugabe von 10.32 g (0.060 mol) p-Toloulsulfonsäure 8 h zum Rückfluß erhitzt (66-67°C). Man destilliert bei Normaldruck den Überschuß Methanol ab, dann unter Vakuum Rest-MeOH und das noch vorhandene Wasser und erhält eine gelbliche trübe Schmelze, die beim Abkühlen zu einer gelblichen Kristallmasse erstarrt. Der Fortgang der Reaktion kann dünnschichtchromatographisch verfolgt werden (Produkt: mehrere Flecken, deutlich Ausgangsmaterial LGS vorhanden).
- Ausbeute:: 116 g
- Reinheit:: ca. 60 % N-Lauroyl-L-glutaminsäuredimethylester (HPLC, FI.%).

## Patentansprüche

1. Verfahren zur Herstellung von N-Lauroyl-L-glutaminsäuredimethylester durch Umsetzung von N-Lauroyl-L-glutaminsäure mit Methanol, dadurch gekennzeichnet, daß in Gegenwart von Orthoameisensäuretrimethylester unter Säurekatalyse gearbeitet wird.

2. Verfahren gemäß Anspruch 1, dadurch gekennzeichnet, daß als saurer Katalysator p-Toluolsulfonsäure verwendet wird.

3. Verfahren gemäß Anspruch 1 und/oder 2, dadurch gekennzeichnet, daß entstandener Ameisensäuremethylester destillativ aus der Reaktionsmischung entfernt wird.

4. Verfahren gemäß einem oder mehreren der Ansprüche 1 bis 3, dadurch gekennzeichnet, daß das aus Orthoameisensäuretrimethylester gebildete Methanol zur Veresterung von N-Lauroyl-L-glutaminsäure mitverwendet wird.

## Claims

1. A process for the preparation of dimethyl N-lauroyl-L-glutamate by reaction of N-lauroyl-L-glutamic acid with methanol, which comprises carrying out the reaction in the presence of trimethyl orthoformate with acid catalysis.

2. The process as claimed in claim 1, wherein the acid catalyst used is p-toluenesulfonic acid.

3. The process as claimed in claim 1 and/or 2, wherein resultant methyl formate is removed from the reaction mixture by distillation.

4. The process as claimed in one or more of claims 1 to 3, wherein the methanol formed from trimethyl orthoformate is additionally used for the esterification of N-lauroyl-L-glutamic acid.

## Revendications

1. Procédé pour la préparation de N-lauroyl-L-glutamate de diméthyle par réaction d'acide N-lauroyl-L-glutamique avec du méthanol, caractérisé en ce qu'on travaille sous catalyse acide en présence d'orthoformiate de triméthyle.

2. Procédé selon la revendication 1, caractérisé en ce qu'on utilise comme catalyseur acide l'acide p-toluène-sulfonique.

3. Procédé selon la revendication 1 et/ou 2, caractérisé en ce que le formiate de méthyle produit est éliminé du mélange réactionnel par distillation.

4. Procédé selon une ou plusieurs des revendications 1 à 3, caractérisé en ce que le méthanol formé à partir de l'orthoformiate de triméthyle participe à l'estérification de l'acide N-lauroyl-L-glutamique.
